# EUROPEAN PATENT APPLICATION

(11) **EP 1 656 939 A1**
(43) Date of publication of application: **17.05.2006**
(21) Application number: 04078099.1
(22) Date of filing: 10.11.2004
(51) Int. Cl.: A61K 31/4045, A61K 45/06, A61P 35/00, A61P 35/04

(54) **Use of melatonin in the manufacture of a medicament for treating cancer**

(71) Applicant: Pooger Properties Limited, 1012 NW Amsterdam (NL)
(72) Inventor: Kruisinga, Roelof Johannes Hendrik, Dr., 2243 HW Wassenaar (NL)
(74) Representative: Huygens, Arthur Victor

(57) **Abstract**

The present invention relates to the use of at least one of melatonin, a melatonin analogue, or a pharmaceutically acceptable salt thereof in the treatment of certain cancers, in particular metastatic colorectal cancer and metastatic breast cancer in a mammal, especially a human being. This treatment is generally associated with one or more of the following characteristics:
- Improvements in quality of life
- Normalized circadian rhythm in serum cortisol

## Description

### Field of the invention

The present invention relates to the use of melatonin in the treatment of certain cancers, in particular metastatic colorectal cancer and metastatic breast cancer, which will normalize the circadian structure of the patients and control the development and progression of cancer and contribute to improve the quality of life and increase the survival time of patients suffering from such disease.

### Background of the invention and related art

### Circadian rhythms

In mammals, most physiological, biochemical and behavioral processes vary in a regular and predictable periodic manner with respect to time of day (endogenous circadian rhythm) (1). The suprachiasmatic nuclei (SCN) of the anterior hypothalamus are the site of the circadian pacemaker in mammals (2). The SCN coordinate the circadian rhythm and through their input pathways from the retina allow synchronization to changes in the sleep/activity routine (3). The output pathways by which the SCN pacemaker organizes daily rhythms in peripheral tissues involve both the autonomic nervous system and the neuroendocrine system (4-6).

### Cancer and abnormal circadian rhythms

Cancer can alter circadian function in both experimental tumor models and in patients (7). Abnormalities in the cortisol rhythm, melatonin rhythm and sleep-activity rhythm have been implicated in cancer development and progression. An in-depth study of such abnormalities forms the basis of the present invention.

### Melatonin

Melatonin (N-acetyl-5-methoxytryptamine) is an endogenous hormone of the pineal gland, a small organ (approx. 100 mg) located in the mid-brain above the third ventricle (8). The rate-limiting enzyme for its synthesis, N-acetyltransferase (NAT) is produced only during the night. Night-time values of NAT are more than 100-fold greater than daytime levels. Melatonin is also produced by extra-pineal tissues like the retina and the gut. Melatonin has been postulated as the mediator of photic-induced antigonadotropic activity in photoperiodic mammals and has also been shown to be involved in thermoregulation in some ectotherms and in affecting locomotor activity rhythms in sparrows.

Melatonin, when used experimentally, is synthesised chemically and has been studied extensively in clinical and preclinical trials to examine the effects of the circadian SCN clock (9).

### Summary of the invention

In contrast to earlier reports that application of melatonin may cause an improvement of the quality of life in patients suffering from metastatic colorectal cancer and metastatic breast cancer it has now surprisingly been found that this improvement of the quality of life is not due to an oncostatic effect of melatonin or an antioxydant effect, but to a re-adjustment of the circadian organization by application of a low dose of melatonin in the evening. This will induce a normalization of the serum cortisol rhythm. The normalizations of the melatonin rhythm as well as the cortisol rhythm will in its turn readjust the circadian organization by resetting the circadian pacemaker of the patient which will not only result in an improvement of the quality of life (as measured by the EORTC QLQ-C-30 (ref. 10)), but also with an increase in survival time of the patient.

Accordingly, the present invention provides in one aspect the use of at least one of melatonin, a melatonin analogue, or a pharmaceutically acceptable salt of melatonin or said melatonin analogue, in the preparation of a medicament for controlling the development and progression of cancer, in particular, but not limited to, metastatic colorectal cancer and metastatic breast cancer in patients suffering from such disease.

It has also been found that the timing and dose of the administration of the melatonin composition are quite essential. For the regulation of the circadian control the moment of administration is preferably at the end of the activity phase. It has also been found that low dosages are preferred for administration of the medicament according to the invention. Suitable low dosages are ranging in an amount of from 0.005 to 10.00 mg/kg, more preferably in an amount of about 0.05 mg to about 5.00 mg per dose.

In a preferred embodiment of the invention the medicament is formulated as a controlled release preparation.

In another preferred embodiment the medicament is formulated as a solid oral formulation.

The medicament may additionally contain one or more substances selected from the group of stimulants, hormones, analogues of such hormones, phyto-hormones, analogues of such phyto-hormones, and anti-oxidants.

In another aspect of the invention a method is provided of treating certain cancers in a mammal, in particular a human, to promote one or more characteristics selected from the group of improving quality of life, normalizing circadian rhythm in serum cortisol, which comprises administering to said mammal a therapeutically effective amount of melatonin, a melatonin analogue, or a pharmaceutically acceptable salt of melatonin or said melatonin analogue.

These and other aspects of the invention will be described in more detail in the following detailed description.

### Detailed description of the invention

### Definition

As used herein, unless stated otherwise, the term "melatonin" includes melatonin per se, a melatonin analogue, i.e. a substance exhibiting high affinity for melatonin receptors, or a pharmaceutically acceptable salt of melatonin or a melatonin analogue.

### The sleep-activity rhythm

In man, the sleep-activity rhythm can be used as a marker of the endogenous circadian clock function in health and disease (11-13). The sleep-activity rhythm was monitored by actigraphy in 192 patients with metastatic colorectal cancer before starting chemotherapy in a prospective study (14). Patients wore a small wrist-watch (actigraph), which records the number of arm movements per minute, for 3 days before receiving chemotherapy.

A near normal circadian rhythm in sleep-activity predicted for a better response and longer survival in both a univariate and a multivariate Cox analysis. Survival at 2 years was 5-fold higher (P = 0.0001) in patients with near normal activity rhythm when compared to patients with rhythm alteration. Patients with near normal sleep/activity rhythms had a better quality of life (EORTC QLQ-C30) and reported significantly less fatigue. The estimate of cortisol circadian rhythm was positively correlated with r24. The individual sleep/activity cycle therefore provides a novel independent prognostic factor for survival and response as well as a quantitative measure of quality of life.

Actigraphy has also been used to study patient with metastatic breast cancer. Data were collected as a part of a placebo controlled randomized study examining the efficacy of paroxetine in attenuating or preventing the development of fatigue during chemotherapy (15). Actigraphy data were collected for 3 days at baseline for 78 patients after receiving 2 courses of CMF (37%), CAP (45%) or other (18%) chemotherapy. The autocorrelation coefficients (r24) at baseline had a normal distribution and ranged from 0.16 to 0.85 (median=0.56). For comparison, the r24 at baseline in the 192-colorectal cancer patients described above (14) ranged from -0.06 to 0.77 (median 0.42). Actigraphy data were obtained for 45 (58%) of these patients after their fourth cycle of chemotherapy. Changes in the fatigue, depression and mood measures, during 2 months of chemotherapy, were significantly correlated with concurrent positive or negative changes in actigraphy parameters (r24). This data show that Actigraphy is a valid and reliable means of assessing fatigue in cancer patients and is sensitive to changes in these parameters over a month period.

Also in animals there is a relation between circadian rhythmicity and tumor growth. In rodents, the destruction of the SCN suppresses the sleep-activity rhythm, while fetal suprachiasmatic transplants restores it (16-18). Mutations of genes involved in circadian regulation also induce profound alterations of the sleep-activity cycle in hamsters and mice (18, 19). Mice with normal or lesioned SCN were inoculated with implants of Glasgow osteosarcoma or pancreatic adenocarcinoma tumors to determine the effects of altered circadian rhythms on tumor progression (20). In the SCN lesioned mice, the sleepactivity rhythms and cortisol rhythms were markedly altered. Both tumors types grew 2-3 times faster in mice with SCN lesions than in sham-operated mice (two-way ANOVA: P < 0.001) and survival was significantly shorter (log-rank P = 0.0062).

In a subsequent study, the consequence of experimental jet lag on circadian rhythm and on tumor growth in mice was investigated (21). Experimental jet lag, produced by an 8-hour advance of the light-dark cycle every 2 days, resulted in an altered sleepactivity rhythm. Tumor grew faster in the jet-lagged animals (p from ANOVA <0.001) and survival was significantly shorter (log-rank P = 0.013). This study confirms the findings in an earlier report where alternating the photoperiod between LD 14:10 and DL 10:14 every 3 days disrupted the circadian rhythm in Ehrlich-carcinoma- and sarcoma-180-bearing mice. The associated reduction of survival and acceleration of tumor growth was reversed by melatonin therapy (22).

Taken together these data suggest that malignant tumors can still response to signals from the SCN and that a disruption of circadian clock coordination in malignant tumors can play a significant role in tumor progression. Therapeutic strategies aimed at normalizing circadian clock dysfunction may impact on the circadian rhythm in malignant tumors with a favorable impact on cancer progression. The poorer survival of cancer patients with abnormal circadian rhythms is not just a reflection of their cancer burden or performance status, but directly related to their abnormal circadian rhythm and the associated molecular abnormalities important for the control of cell cycle, apoptosis and tumor suppression.

### The cortisol rhythm

In healthy individuals, cortisol levels have a circadian rhythm; high in the morning and low at night (23). Abnormalities in the cortisol rhythm have been implicated in tumor growth in animals and cancer cell line studies (24,25), and can have profound impact on the immune system 26,27).

Studies in breast cancer patients have found that abnormalities in the diurnal cortisol rhythm are common (28,29) and associated with a poor performance status and liver metastases (26).

The above data in breast cancer patients suggest that abnormal circadian rhythms are not simply a reflection of preterminal changes, but a relatively long-term prognostic indicator.

### The melatonin rhythm

Melatonin is secreted by the pineal gland in a circadian manner (30,31), reaching a peak early in the morning (2 a.m. to 4 a.m.). When humans are exposed to light, neural input from the SCN inhibits pineal activity and thus melatonin synthesis (32). In the dark phase the opposite situation exists, with nocturnal blood concentrations being approximately ten fold higher than those observed during the day.

Three recent studies on women who routinely worked night shifts suggest that light expose at night, with the associated disruption of the melatonin rhythm, may be a risk factor for breast cancer (33-35). All studies adjusted their outcomes for known confounders.

Positive associations between shift-work and breast cancer risk were also observed in three smaller cohort studies (36-38) conducted for other purposes, but these studies did not adjust for other confounders for breast cancer. Finally, indirect support comes from studies on blind women who are not sensitive to changes in light and, consequently, whose melatonin levels do not change. These studies show an approximately 20%-50% reduced risk of breast cancer among such women (39-42) with a tendency toward an inverse dose-response relationship between the degree of blindness and breast cancer risk (43).

Several mechanisms involved in the apparent protective effect of melatonin have been suggested including; a direct antiproliferative effect (44), increased immune responses (30,45) scavenging of free radicals (89), and modulation of the expression of the tumor suppressor gene p53 (46). Melatonin has been shown to have significant antitumor activity in rodents (47); pinealectomy increases tumor growth (48), melatonin inhibits the promotion of chemically induced mammary tumors (48,49), and constant light exposure has a growth-promoting effect on chemically induced tumors (50). In a series of studies, Blask et al. have found that melatonin inhibits tumor growth via a melatonin receptor-mediated suppression of cAMP levels resulting in decreased tumor transport and metabolism of linoleic acid to 13-hydroxyoctadecadienoic acid (51-53).

The medicament for the treatment of certain cancers according to the present invention is suitably administered to the mammal in the form of a pharmaceutical composition. The administration may be by way of oral or parenteral administration.

The medicament can be administered in conventional form for oral administration, e.g. as tablets, lozenges, dragees and capsules. In certain cases, for example when the drug is administered to children, it may be preferred to formulate the composition as an oral liquid preparation such as a syrup, a nasal spray, or a suppository. The medicament can also be administered parenterally, e.g. by intramuscular or subcutaneous injection, using formulations in which the medicament is employed in a saline or other pharmaceutically acceptable, injectable composition.

An amount effective to treat the disease hereinbefore described depends on the usual factors such as the nature and severity of the disorder being treated, the weight of the mammal, the specific compound(s) of choice, and considerations and preferences of the prescriber. The amount of active ingredient(s) to be administered usually will be in the range of nanograms to 50 mg or more per dose. However, a unit dose will normally contain 1 to 1000 mg, suitably 1 to 500 mg, for example an amount in the range of from 2 to 400 mg such as 2, 5, 10, 20, 30, 40, 50, 100, 200, 300 and 400 mg of the active ingredient. Unit doses will normally be administered once or more than once per day, for example 1, 2, 3, 4, 5 or 6 times a day, more usually 1 to 4 times a day, such that the total daily dose is normally in the range, for a 70 kg adult, of 1 to 1000 mg, for example 1 to 500 mg, that is in the range of approximately 0.01 to 15 mg/kg/day, more usually 0.1 to 6 mg/kg/day, for example 1 to 6 mg/kg/day.

It is greatly preferred that melatonin is administered in the form of a unit-dose composition, such as a unit dose oral, such as sub-lingual, rectal, topical or parenteral (especially intravenous) composition.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use. The preparation of such compositions is well known to people skilled in the art and can be optimized in a routine way without exerting inventive skill and without undue experimentation.

Tablets and capsules for oral administration are usually presented in a unit dose and contain conventional excipients such as binding agents, fillers, diluents, tabletting agents, lubricants, disintegrants, colourants, flavourings, and wetting agents. The tablets may be coated according to well known methods in the art.

Suitable fillers for use include, mannitol and other similar agents. Suitable disintegrants include starch derivatives such as sodium starch glycollate. Suitable lubricants include, for example, magnesium stearate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral formulations further include controlled release formulations which may also be useful in the practice of this invention. The controlled release formulation may be designed to give an initial high dose of the active material and then a steady dose over an extended period of time, or a slow build up to the desired dose rate, or variations of these procedures. Controlled release formulations also include conventional sustained release formulations, for example tablets or granules having an enteric coating.

Nasal spray compositions are also a useful way of administering the pharmaceutical preparations of this invention to patients for whom compliance is difficult. Such formulations are generally aqueous and are packaged in a nasal spray applicator which delivers a fine spray of the composition to the nasal passages.

Suppositories are also a traditionally good way of administering drugs to children and can be used for the purposes of this invention. Typical bases for formulating suppositories include water-soluble diluents such as polyalkylene glycols and fats, e.g. cocoa oil and polyglycol ester or mixtures of such materials.

For parenteral administration, fluid unit dose forms are prepared containing melatonin and a sterile vehicle. The melatonin, depending on the vehicle and the concentration, can be either suspended or dissolved. Parenteral solutions are normally prepared by dissolving the melatonin in a vehicle and filter sterilising before filling into a suitable vial or ampoule and sealing. Advantageously, adjuvants such as a local anaesthetic, preservatives and buffering agents are also dissolved in the vehicle.

Parenteral suspensions are prepared in substantially the same manner except that the melatonin is suspended in the vehicle instead of being dissolved and sterilised usually by exposure to ethylene oxide before suspending in the sterile vehicle. Advantageously, a surfactant or wetting agent is included in the composition to facilitate uniform distribution of the melatonin.

As is common practice, the compositions will usually be accompanied by written or printed directions for use in the medical treatment concerned.

The present invention further provides a pharmaceutical composition comprising at least one of melatonin, a melatonin analogue, or a pharmaceutically acceptable salt of melatonin or said melatonin analogue, and a pharmaceutically acceptable carrier. These pharmaceutical compositions may be prepared in a manner known per se, such as the manner as described hereinbefore.

In the treatment of patients with certain cancers in accordance with the invention, in particular metastatic colorectal cancer and metastatic breast cancer, melatonin can be used alone or together with other active materials. The latter materials are preferably chosen such that either their activity is enhanced, preferably in a synergistic way, or undesired side-effects are suppressed by melatonin and/or its analogue. For example, the melatonin which can be used in conjunction with another active material additionally contains one or more substances selected from the group of stimulants, hormones, analogues of such hormones, phyto-hormones, analogues of such phyto-hormones like phyto estrogen, and anti-oxidants like phyto-vitamins C and E, flavonoids.

The recommended dosages of melatonin and, optionally, other active ingredients in the use according to the present invention are either documented in the art for its/their use in other medicaments or can be determined experimentally without inventive skill.

Preliminary investigations show the following dose rates for melatonin. For the occasional self-treatment of mild insomnia in adults: 0.3 to 3 mg oral or sublingual dosage (PO), in the evening hours approximately 1 to 2 hours before habitual bedtime. May take up to 6 mg PO if needed. For the adjunctive treatment of insomnia related to major depression: Adults: 5 to 10 mg oral extended release formulations (PO) taken 1 to 2 hours prior to habitual bedtime. In one 4-week placebo-controlled study of 19 patients with major depressive disorder treated with fluoxetine, the sub-group of 10 patients who received concomitant slow-release melatonin at 9 pm for sleep reported significantly improved sleep quality scores versus the patients receiving fluoxetine alone. Melatonin treatment avoided the need for additional sleep medications. No differences in the rates of improvement of depressive symptoms or side effects were reported between the two groups. (Dolberg et al; 1998)

### Melatonin Pharmacokinetics

The half-life of endogenous melatonin in the serum ranges from 30-50 minutes. Approximately 90% of melatonin is cleared in a single passage through the liver, some of which may be excreted into the bile. Microsomal enzymes in hepatic cells metabolize melatonin to 6-hydroxymelatonin. The latter product is subsequently conjugated with sulfate or to glucuronide and excreted in the urine (31).
Oral administration of 0.3 mg of melatonin in humans during the day elevates plasma concentrations to a level comparable with those of normal nocturnal values (54). Melatonin is absorbed rapidly. When given orally, plasma levels increase strikingly after 30 to 150 minutes, with an elimination half-life of 0.5 to 0.8 hours (55, 56).

### Melatonin Toxicity

In human volunteers no adverse side effects have been observed after oral administration of melatonin in daily doses of 1 to 300 mg (57-65). Doses as high as 1000 mg daily have been safely administered for 30 days (66). A randomized double-blind clinical trial specifically examined melatonin for potential toxicologic effects in healthy adult male subjects (67). The investigators gave 10 mg melatonin daily for 28 days and reported no evidence of toxicity.

The following Examples show some useful pharmaceutical formulations of melatonin in the treatment of certain cancers according to the present invention.

### Example 1

A tablet is formulated containing:

| | |
|---|---|
| Melatonin | 5.0 mg |
| Mannitol | 20.0 mg |
| Calcium hydrogen phosphate | 42.0 mg |
| Sodium starch glycollate | 5.0 mg |
| Talc | 2.5 mg |
| Magnesium stearate | 0.5 mg |

The dissolution profile of this tablet results in a melatonin release of more than 90% within 30 minutes. The disintegration time is very short and the materials meet the requirements for a dispersible tablet (Ph. Eur).

### Example 2

A capsule is formulated containing:

| | |
|---|---|
| Melatonin | 5.0 mg |
| Mannitol | 20.0 mg |
| Calcium hydrogen phosphate | 66.0 mg |
| Ethylcellulose | 1.0 mg |
| Sodium starch glycollate | 5.0 mg |
| Talc | 2.5 mg |
| Magnesium stearate | 0.5 mg |
| HPMC Capsule | 37.5 mg |

The dissolutie profile of this capsule results in a direct release of 1.8 mg of melatonin (90% of 2 mg) within 30 minutes, and a sustained release of the remaining 3 mg within 6 hours.

### References

1. Pittendrigh CS. Temporal organization: reflections of a Darwinian clock-watcher. Annu Rev Physiol 1993;55:16-54.
2. Weaver DR. The suprachiasmatic nucleus: a 25-year retrospective. J Biol Rhythms 1998;13(2):100-12.
3. Meijer JH, Rietveld WJ. Neurophysiology of the suprachiasmatic circadian pacemaker in rodents. Physiol Rev 1989;69(3):671-707.
4. Bartness TJ, Song CK, Demas GE. SCN efferents to peripheral tissues: implications for biological rhythms. J Biol Rhythms 2001; 16(3): 196-204.
5. Kalsbeek A, Buijs RM. Output pathways of the mammalian suprachiasmatic nucleus: coding circadian time by transmitter selection and specific targeting. Cell Tissue Res 2002;309(1):109-18.
6. Buijs RM, Wortel J, Van Heerikhuize JJ, Feenstra MG, Ter Horst GJ, Romijn HJ, Kalsbeek A. Anatomical and functional demonstration of a multisynaptic suprachiasmatic nucleus adrenal (cortex) pathway. Eur J Neurosci 1999;11 (5):1535-44.
7. Mormont MC, Levi F. Circadian-system alterations during cancer processes: a review. lnt J Cancer 1997;70(2):241-7.
8. Lerner AB et al., J. Amer. Chem. Soc. 1958; 80:2587
9. Lewy AJ et al., Behav. Brain Res. 1996; 73:1-2 131-4
10. Aaronson NK, Ahmedzai S, Bergman B, Bullinger M, Cull A, Duez NJ, Filiberti A, Flechtner H, Fleishman SB, de Haes JC, et al. The European Organization for Research and Treatment of Cancer QLQ-C30: a quality-of-life instrument for use in international clinical trials in oncology. J Natl Cancer Inst 1993;85(5):365-76.
11. Wever R. The circadian multi-oscillatory system of man. Int J Chronobiol 1975;3(1):19-55.
12. Duffy JF, Kronauer RE, Czeisler CA. Phase-shifting human circadian rhythms: influence of sleep timing, social contact and light exposure. J Physiol 1996;495(Pt 1):289-97.
13. Moore-Ede MC, Czeisler CA, Richardson GS. Circadian timekeeping in health and disease. Part 2. Clinical implications of circadian rhythmicity. New Engl J Med 1983;309(9):530-536.
14. Mormont MC, Waterhouse J, Bleuzen P, Giacchetti S, Jami A, Bogdan A, Lellouch J, Misset JL, Touitou Y, Levi F. Marked 24-h rest/activity rhythms are associated with better quality of life, better response, and longer survival in patients with metastatic colorectal cancer and good performance status. Clin Cancer Res 2000;6(8):3038-45.
15. Roscoe JA, Morrow GR, Hickok JT, Bushunow P, Matteson S, Rakita D, Andrews PL. Temporal interrelationships among fatigue, circadian rhythm and depression in breast cancer patients undergoing chemotherapy treatment. Support Care Cancer 2002;10(4):329-36.
16. Stephan FK, Zucker I. Circadian rhythms in drinking behavior and locomotor activity of rats are eliminated by hypothalamic lesions. Proc Nat Acad Sci USA 1972;69(6):1583-1586.
17. Drucker-Colin R, Aguilar-Roblero F, Garcia-Hernandez F, Fernandez-Cancinoa F, Rattoni FB. Fetal suprachiasmatic nucleus transplants: diurnal rhythm recovery of lesioned rats. Brain Res 1984;311:353-357.
18. Ralph MR, Foster RG, Davis FC, Menaker M. Transplanted suprachiasmatic nucleus determines circadian period. Science 1990;247:975-978.
19. King DP, Zhao Y, Sangoram AM, Wilsbacher LD, Tanaka M, Antoch MP, Steeves TD, Vitaterna MH, Kornhauser JM, Lowrey PL, Turek FW, Takahashi JS. Positional cloning of the mouse circadian clock gene. Cell 1997;89(4):641-53.
20. Filipski E, King VM, Li X, Granda TG, Mormont MC, Liu X, Claustrat B, Hastings MH, Levi F. Host circadian clock as a control point in tumor progression. J Natl Cancer Inst 2002;94(9):690-7.
21. Filipski E, King VM, Sun J, Hastings MH, Levi F. Experimental jet lag accelerates malignant growth and shortens survival in tumor-bearing mice. Am Assoc Cancer Res. 2002; 43:A4270. (abstract.)
22. Li JC, Xu F. Influences of light-dark shifting on the immune system, tumor growth and life span of rats, mice and fruit flies as well as on the counteraction of melatonin. Biol Signals 1997;6(2):77-89.
23. Posener JA, Schildkraut JJ, Samson JA, Schatzberg AF. Diurnal variation of plasma cortisol and homovanillic acid in healthy subjects. Psychoneuroendocrinology 1996;21 (1):33-8.
24. Sapolsky RM, Donnelly TM. Vulnerability to stress-induced tumor growth increases with age in rats: role of glucocorticoids. Endocrinology 1985;117(2):662-6.
25. Lointier P, Wildrick DM, Boman BM. The effects of steroid hormones on a human colon cancer cell line in vitro. Anticancer Res 1992;12(4):1327-30.
26. Touitou Y, Levi F, Bogdan A, Benavides M, Bailleul F, Misset JL. Rhythm alteration in patients with metastatic breast cancer and poor prognostic factors. J Cancer Res Clin Oncol 1995;121 (3):181-8.
27. Abrahamsen JF, Smaaland R, Sandberg S, Aakvaag A, Lote K. Circadian variation in serum cortisol and circulating neutrophils are markers for circadian variation of bone marrow proliferation in cancer patients. Eur J Haematol 1993;50(4):206-12.
28. Touitou Y, Bogdan A, Levi F, Benavides M, Auzeby A. Disruption of the circadian patterns of serum cortisol in breast and ovarian cancer patients: relationships with tumour marker antigens. Br J Cancer 1996;74(8):1248-52.
29. Singh R, Singh RK, Mahdi AA, Misra S, Rai SP, Singh D, Cornelissen G, Halberg F. Studies on circadian periodicity of urinary corticoids in carcinoma of the breast. In Vivo 1998;12(1):69-73.
30. Brzezinski A. Melatonin in humans. New England Journal of Medicine 1997; 336(3):186-95.
31. Vijayalaxmi, Thomas CR, Jr., Reiter RJ, Herman TS. Melatonin: from basic research to cancer treatment clinics. J Clin Oncol 2002;20(10):2575-601.
32. Lewy AJ, Wehr TA, Goodwin FK. Light suppresses melatonin secretion in human. Science 1980;210:1267-1269.
33. Davis S, Mirick DK, Stevens RG. Night shift work, light at night, and risk of breast cancer. J Natl Cancer Inst 2001;93(20):1557-62.
34. Schernhammer ES, Laden F, Speizer FE, Willett WC, Hunter DJ, Kawachi I, Colditz GA. Rotating night shifts and risk of breast cancer in women participating in the nurses' health study. J Natl Cancer Inst 2001;93(20):1563-8.
35. Hansen J. Increased breast cancer risk among women who work predominantly at night. Epidemiology 2001;12(1):74-7.
36. Pukkala E, Auvinen A, Wahlberg G. Incidence of cancer among Finnish airline cabin attendants, 1967-92. Bmj 1995;311(7006):649-52.
37. Tynes T, Hannevik M, Andersen A, Vistnes Al , Haldorsen T. Incidence of breast cancer in Norwegian female radio and telegraph operators. Cancer Causes Control 1996;7(2):197-204.
38. Rafnsson V, Tulinius H, Jonasson JG, Hrafnkelsson J. Risk of breast cancer in female flight attendants: a population-based study (Iceland). Cancer Causes Control 2001;12(2):95-101.
39. Feychting M, Osterlund B, Ahlbom A. Reduced cancer incidence among the blind. Epidemiology 1998;9(5):490-4.
40. Coleman MP, Reiter RJ. Breast cancer, blindness and melatonin. Eur J Cancer 1992;28(2-3):501-3.
41. Hahn RA. Profound bilateral blindness and the incidence of breast cancer. Epidemiology 1991;2(3):208-10.
42. Kliukiene J, Tynes T, Andersen A. Risk of breast cancer among Norwegian women with visual impairment. Br J Cancer 2001 ;84(3):397-9.
43. Verkasalo PK, Pukkala E, Stevens RG, Ojamo M, Rudanko SL. Inverse association between breast cancer incidence and degree of visual impairment in Finland. Br J Cancer 1999;80(9):1459-60.
44. Baldwin WS, Barrett JC. Melatonin: receptor-mediated events that may affect breast and other steroid hormone-dependent cancers. Mol Carcinog 1998;21(3):149-55.
45. Maestroni GJ. MLT and the immune-hematopoietic system. Adv Exp Med Biol 1999;460:395-405.
46. Mediavilla MD, Cos S, Sanchez-Barcelo EJ. Melatonin increases p53 and p21WAF1 expression in MCF-7 human breast cancer cells in vitro. Life Sci 1999;65(4):415-20.
47. Blask DE, Sauer LA, Dauchy R, Holowachuk EW, Ruhoff MS. New actions of melatonin on tumor metabolism and growth. Biol Signals Recept 1999;8(1-2):49-55.
48. Tamarkin L, Cohen M, Roselle D, Reichert C, Lippman M, Chabner B. Melatonin inhibition and pinealectomy enhancement of 7,12-dimethylbenz(a)anthracene-induced mammary tumors in the rat. Cancer Res 1981,41(11 Pt 1):4432-6.
49. Blask DE, Pelletier DB, Hill SM, Lemus-Wilson A, Grosso DS, Wilson ST, Wise ME. Pineal melatonin inhibition of tumor promotion in the N-nitroso-N-methylurea model of mammary carcinogenesis: potential involvement of antiestrogenic mechanisms in vivo. Journal of Cancer Research & Clinical Oncology 1991;117(6):526-32.
50. van den Heiligenberg S, Depres-Brummer P, Barbason H, Claustrat B, Reynes M, Levi F. The tumor promoting effect of constant light exposure on diethylnitrosamine- induced hepatocarcinogenesis in rats. Life Sci 1999;64(26):2523-34.
51. Dauchy RT, Blask DE, Sauer LA, Brainard GC, Krause JA. Dim light during darkness stimulates tumor progression by enhancing tumor fatty acid uptake and metabolism. Cancer Lett 1999;144(2):131-6.
52. Dauchy RT, Sauer LA, Blask DE, Vaughan GM. Light contamination during the dark phase in "photoperiodically controlled" animal rooms: effect on tumor growth and metabolism in rats. Lab Anim Sci 1997;47(5):511-8.
53. Blask DE, Sauer LA, Dauchy RT, Holowachuk EW, Ruhoff MS, Kopff HS. Melatonin inhibition of cancer growth in vivo involves suppression of tumor fatty acid metabolism via melatonin receptor-mediated signal transduction events. Cancer Res 1999;59(18):4693-701.
54. Zhdanova IV, Wurtman RJ, Morabito C, Piotrovska VR, Lynch HJ. Effects of low oral doses of melatonin, given 2-4 hours before habitual bedtime, on sleep in normal young humans. Sleep 1996;19(5):423-31.
55. Waldhauser F, Waldhauser M, Lieberman HR, Deng MH, Lynch HJ, Wurtman RJ. Bioavailability of oral melatonin in humans. Neuroendocrinology 1984;39(4):307-13.
56. Aldhous M, Franey C, Wright J, Arendt J. Plasma concentrations of melatonin in man following oral absorption of different preparations. Br J Clin Pharmacol1985;19(4):517-21.
57. Lieberman HR, Waldhauser F, Garfield G, Lynch HJ, Wurtman RJ. Effects of melatonin on human mood and performance. Brain Res 1984;323(2):201-7.
58. Arendt J, Aldhous M, Marks V. Alleviation of jet lag by melatonin: preliminary results of controlled double blind trial. Br Med J (Clin Res Ed) 1986;292(6529):1170.
59. Lissoni P, Barni S, Crispino S, Tancini G, Fraschini F. Endocrine and immune effects of melatonin therapy in metastatic cancer patients. European Journal of Cancer & Clinical Oncology 1989;25(5):789-95.
60. James SP, Sack DA, Rosenthal NE, Mendelson WB. Melatonin administration in insomnia. Neuropsychopharmacology 1990;3(1):19-23.
61. Samel A, Wegmann HM, Vejvoda M, Maass H, Gundel A, Schutz M. Influence of melatonin treatment on human circadian rhythmicity before and after a simulated 9-hr time shift. J Biol Rhythms 1991;6(3):235-48.
62. Voordouw BC, Euser R, Verdonk RE, Alberda BT, de Jong FH, Drogendijk AC, Fauser BC, Cohen M. Melatonin and melatonin-progestin combinations alter pituitary-ovarian function in women and can inhibit ovulation. J Clin Endocrinol Metab 1992;74(1): 108-17.
63. Lissoni P, Barni S, Rovelli F, Brivio F, Ardizzoia A, Tancini G, Conti A, Maestroni GJ. Neuroimmunotherapy of advanced solid neoplasms with single evening subcutaneous injection of low-dose interleukin-2 and melatonin: preliminary results. European Journal of Cancer 1993;29A(2): 185-9.
64. Vijayalaxmi, Reiter RJ, Herman TS, Meltz ML. Melatonin and radioprotection from genetic damage: in vivo/in vitro studies with human volunteers. Mutat Res 1996;371 (3-4):221-8.
65. Vijayalaxmi, Reiter RJ, Herman TS, Meltz ML. Melatonin reduces gamma radiation- induced primary DNA damage in human blood lymphocytes. Mutat Res 1998;397(2):203-8.
66. Norlund JJ, Lerner AB. The effects of oral melatonin on skin color and on the release of pituitary hormones. J Clin Endocrinol Metab 1977;45:768-774.
67. Seabra ML, Bignotto M, Pinto LR, Jr., Tufik S. Randomized, double-blind clinical trial, controlled with placebo, of the toxicology of chronic melatonin treatment. J Pineal Res 2000;29(4): 193-200.

## Claims

1. Use of at least one of melatonin, a melatonin analogue, or a pharmaceutically acceptable salt of melatonin or said melatonin analogue, in the preparation of a medicament for controlling the development and progression of cancer, in particular, but not limited to, metastatic colorectal cancer and metastatic breast cancer in patients suffering from such disease.

2. Use as claimed in claim 1 wherein said treatment is associated with one or more of the following characteristics:
• Improvements in quality of life
• Normalized circadian rhythm in serum cortisol

3. Use as claimed in claim 1 or claim 2 wherein the melatonin or melatonin analogue is employed in an amount of from 0.005 to 10.00 mg/kg, in particular 0.05 to 5.00 mg/kg.

4. Use as claimed in any one of the preceding claims wherein the medicament is formulated as a controlled release preparation.

5. Use as claimed in any one of the preceding claims wherein the medicament is formulated as a solid oral formulation.

6. Use as claimed in any one of the preceding claims wherein the medicament additionally contains one or more substances selected from the group of stimulants, hormones, analogues of such hormones, phyto-hormones, analogues of such phyto-hormones, and anti-oxidants.

7. A method of treating certain cancers in a mammal, in particular a human, to promote one or more characteristics selected from the group of improving quality of life, normalizing circadian rhythm in serum cortisol and norm, which comprises administering to said mammal a therapeutically effective amount of melatonin, a melatonin analogue, or a pharmaceutically acceptable salt of melatonin or said melatonin analogue.
